# EUROPEAN PATENT SPECIFICATION

(11) **EP 3 079 672 B1**
(45) Date of publication and mention of the grant of the patent: **13.05.2020**
(21) Application number: 14815635.9
(22) Date of filing: 11.12.2014
(51) Int. Cl.: A61K 9/20, A61K 31/135

(54) **PHARMACEUTICAL COMPOSITION COMPRISING A PHARMACEUTICALLY ACCEPTABLE SALT OF RASAGILINE**
PHARMAZEUTISCHE ZUSAMMENSETZUNG MIT EINEM PHARMAZEUTISCH AKZEPTABLEN SALZ AUS RASAGILIN
COMPOSITION PHARMACEUTIQUE COMPRENANT UN SEL PHARMACEUTIQUEMENT ACCEPTABLE DE RASAGILINE

(30) Priority: 11.12.2013 SI 201300418
(43) Date of publication of application: 19.10.2016
(73) Proprietor: KRKA, d.d., Novo mesto, 8000 Novo mesto (SI)
(72) Inventor: CERPNJAK, Katja, 8220 Smarjeske Toplice (SI); KROSELJ, Vesna, 8000 Novo mesto (SI); SKUBE, Maja Kincl, 8000 Novo mesto (SI); KOLENC, Ivanka, 8000 Novo mesto (SI); VRECER, Franc, 8351 Straza pri Novem mestu (SI)
(74) Representative: Uexküll & Stolberg
(86) International application number: PCT/EP2014/077434
(87) International publication number: WO 2015/086768

(56) References cited:
- EP-A2- 2 796 130
- WO-A1-2011/003938
- WO-A1-2013/063082
- WO-A1-2013/139387
- CN-A- 1 911 211
- US-A1- 2010 137 447
- US-A1- 2010 234 636
- US-A1- 2010 234 636
- US-A1- 2011 313 050
- Evonik Industries: "Aerosil- Fumed Silica Technical Overview", internet citation, 1 December 2015 (2015-12-01), XP55447974, Retrieved from the Internet: URL:https://www.aerosil.com/sites/lists/RE /DocumentsSI/Technical-Overview-AEROSIL-Fu med-Silica-EN.pdf [retrieved on 2018-02-05]

## Description

### Field of the invention

The present invention relates to a pharmaceutical composition comprising a pharmaceutically acceptable salt, namely a tartrate salt, of rasagiline exhibiting storage stability. The present invention also relates to a process for preparing the pharmaceutical composition.

### Background of the invention

Rasagiline is a common name for (*R*)-*N*-(prop-2-ynyl)-2,3-dihydro-1*H*-inden-1-amine, a compound of formula (I):

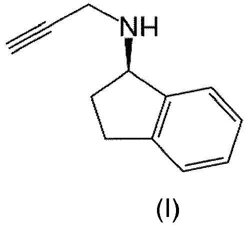

Rasagiline functions as a selective, irreversible MAO-B inhibitor indicated for the treatment of signs and symptoms of idiopathic Parkinson's disease as initial monotherapy and as adjunct therapy to levodopa. The selectivity for inhibiting MAO-B diminishes in a dose-related manner. MAO, a flavin-containing enzyme, is classified into two major molecular species, A and B, and is localized in mitochondrial membranes throughout the body in nerve terminals, brain, liver and intestinal mucosa. MAO regulates the metabolic degradation of catecholamines and serotonin in the CNS and peripheral tissues. MAO-B is the major form in the human brain. In *ex vivo* animal studies in brain, liver and intestinal tissues, rasagiline was shown to be a potent, irreversible monoamine oxidase type B (MAO-B) selective inhibitor. Rasagiline at the recommended therapeutic dose was also shown to be a potent and irreversible inhibitor of MAO-B in platelets. The precise mechanisms of action of rasagiline are unknown. One mechanism is believed to be related to its MAO-B inhibitory activity, which causes an increase in extracellular levels of dopamine in the striatum. The elevated dopamine level and subsequent increased dopaminergic activity are likely to mediate rasagiline's beneficial effects seen in models of dopaminergic motor dysfunction.

Rasagiline is available on the market in the form of a mesylate salt and is sold under trade name Azilect®, as oral tablet in 0.5mg and 1mg strengths. The tablet comprises mannitol, starch, pregelatinized starch, colloidal silicon dioxide, stearic acid and talc as inactive ingredients.

EP 436492 discloses rasagiline, a process for production thereof, its use and a pharmaceutical composition thereof. The patent discloses *inter alia* the composition of tablets comprising active ingredient in admixture with non toxic pharmaceutically acceptable excipients, which may be e.g. inert diluents such as lactose, pregelatinized starch, microcrystalline cellulose, sodium starch glycolate, talc, and magnesium stearate. In the document rasagiline hydrochloride and rasagiline tartrate are specifically mentioned. As a technological process used for the preparation of pharmaceutical compositions wet granulation using water or ethanol has been disclosed.

EP 812190 is related to specific salts of rasagiline selected from mesylate, esylate and sulfate. These salts are said to exhibit superior properties in terms of solubility and chemical stability. Due to its excellent stability, the mesylate salt was selected as most favorable salt.

Alkyl mesylates can be found as potential impurities, in particular in active substances that occur as salts of methanesulfonic acid, i.e. mesylates, or in active substances for which methanesulfonic acid is used in the synthesis. Mesylate esters of C₁-C₃ short chain alcohols are reactive, direct acting, genotoxic and possibly carcinogenic agents, therefore their presence in pharmaceutical compositions should be minimized. As potentially genotoxic impurities can be formed when rasagiline is used in the form of a mesylate salt it was the aim of the present inventors to prepare a pharmaceutical composition comprising rasagiline in the form of a pharmacologically acceptable salt other than the mesylate salt, which salt is stable and pharmacologically equivalent to the mesylate salt.

In the literature there are reported several pharmaceutical compositions comprising rasagiline tartrate.

EP 0858328 discloses a pharmaceutical composition comprising rasagiline or a pharmaceutically acceptable salt thereof, *inter alia* in the form of a tartrate, and at least 60 % by weight of at least one pentahydric or hexahydric alcohol, like mannitol, xylitol or sorbitol. Optionally the composition may contain citric acid and magnesium stearate.

It was observed by the present inventors that tablets prepared according to teaching of EP 858328 comprising rasagiline mesylate or rasagiline tartrate salt exhibit different stability results. Whereas rasagiline mesylate tablet exhibits excellent stability when subjected to accelerated stability testing conditions, on the other hand the tablet comprising rasagiline tartrate does not meet requirements for storage stable formulations. This comparison is described in more detail below in Reference Examples 1 and 2.

WO 2010/070090 discloses a homogeneous mixture containing the active ingredient in a non crystalline form. The active ingredient and excipient are present in the composition in the form of a solid solution in which they are mixed at a molecular level. The solid compositions are prepared by melting of the excipient and active ingredient by methods such as melt extrusion, or by dissolving the excipient and active ingredient in a solvent and then evaporating the solvent. Both, melting and evaporating, generally proceed at higher temperatures. Therefore, the process is not suitable for the drugs which are susceptible for thermal degradation. Rasagiline is known to be prone to thermal degradation and therefore the use of processes where high temperatures are employed should be avoided. The drawbacks of this process are manifold. It is a thermal process leading to problems with drug/polymer stability. Only limited numbers of polymers with desired flow properties are available. Special equipment is required for this process. Moreover, the tableting mass produced by this process might be sticky which results in sticking of the mass to the punches. It was therefore the aim of the present inventors to avoid technologically demanding processes and to use simple and economically acceptable technologies.

WO 2008/019871 discloses a pharmaceutical composition comprising a salt of rasagiline, such as the oxalate or the ethanedisulfonate salt. The composition is prepared by wet granulation with mannitol, by wet granulation without mannitol or by direct compression. Based on a stability study under accelerated conditions it was concluded that most stable compositions are the compositions of rasagiline in the form of the mesylate or the ethanedisulfonate salt. The composition of rasagiline tartrate was found to be the least stable composition irrespective of the method for the preparation used. Considering the teaching of this document a person skilled in the art would be lead away from using rasagiline tartrate for providing a pharmaceutical composition.

WO 2013/107441 and WO 2013/133938 disclose a process for preparation of rasagiline tartrate compositions where the active ingredient is dissolved in the presence of an acid such as tartaric acid, phosphoric acid, benzoic acid or maleic acid, and further mixed with a solid carrier. The presence of an acid is claimed to improve the stability of the composition, however the results of the stability testing presented in these documents cannot confirm that the problem of impurities formation has been actually solved.

There is still a need for a pharmaceutical composition of rasagiline showing a high storage stability, and a high content uniformity, even when the concentration of rasagiline is low. Moreover, the composition should be easily prepared by a simple and cost-saving process.

It was now found out that a desired storage stability, *in vitro* dissolution behavior and processability of pharmaceutical compositions of rasagiline is highly dependent on the technological manufacturing processes used. It was observed that a wet granulation process results in a composition which includes a higher degree of impurities immediately after production, as well as after accelerated stability conditions. Physiochemical properties and forced degradation studies showed that rasagiline has a tendency to produce oxidative, alkaline and acid degradation products in addition to that it is a moisture sensitive drug. The increased surface area, which is a consequence of partial or complete dissolving of the drug during the granulation process, may cause an increase in oxidative degradation of rasagiline and may also influence its interaction with the acid.

Most of the processes for production of pharmaceutical compositions of rasagiline disclosed in the prior art comprises the step of dissolving a rasagiline salt. It has now been found that the drug in the dissolved form is more susceptible to interaction with excipients and to degradation. Accordingly, it has now been found that a process for preparing a pharmaceutical composition of rasagiline is particularly advantageous which would omit all the processes which might induce degradation of rasagiline, such as thermal processes, acidic conditions or dissolving of the drug during technological process.

We have unexpectedly found out that a desired storage stability and a dissolution profile similar to that obtained for marketed product Azilect® can be achieved by using a dry manufacturing process.

### Figures

Figure 1 shows a dissolution profile of the pharmaceutical compositions prepared according to Examples 3 and 4 in 0.1M HCl.
Figure 2 shows a dissolution profile of the pharmaceutical compositions prepared according to Examples 5, 8 and 9 in 0.1M HCl.
Figure 3 shows a dissolution profile of pharmaceutical compositions prepared according to Examples 8 and 9 at pH 6.8.

### Summary of the invention

The present invention is directed to a pharmaceutical composition comprising (a), as the active ingredient, 0.2 to 5%, by weight of the composition, of rasagiline tartrate, (b) diluent, (c) lubricant and/or glidant and optionally (d) disintegrant and (e) binder, wherein the composition is prepared by direct compression, with the direct compression process comprising
- mixing rasagiline tartrate and at least one excipient or a mixture of excipients to form a compression mixture; and
- compressing the compression mixture to a solid dosage form,
wherein the composition is free of antioxidants and acids selected from the group consisting of citric acid, tartaric acid, phosphoric acid, benzoic acid and maleic acid, and wherein the composition comprises a mixture of microcrystalline cellulose and modified starch as diluents and/or binder, wherein the modified starch has an average particle size from 10 µm to 100 µm, wherein the average particle size refers to volume mean diameter determined by laser light scattering.

According to a preferred embodiment of the present invention the pharmaceutical formulation comprises (a) as the active ingredient, rasagiline hemitartrate..

According to another embodiment there is provided a direct compression method for manufacturing the pharmaceutical formulation as defined above which comprises
- mixing rasagiline tartrate and at least one excipient or a mixture of excipients to form a powder mixture which can be optionally sieved before mixing to give a compression mixture;
- compressing the compression mixture to desired solid dosage form;
- optionally, applying a film coating.

According to a preferred embodiment of the present invention, a direct compression process for the manufacturing the pharmaceutical formulation as defined above comprises:
- mixing rasagiline tartrate and a portion of diluent in a weight/weight ratio from 1:5 to 1: 50, preferably from 1:10 to 1:30, more preferably from 1:15 to 1:25, and/or further excipients and sieving the mixture,
- mixing the above obtained mixture with the main portion of diluent and/or further excipients in a mixer to obtain a homogenized mixture,
- optionally dry sieving the mixture through a sieve in order to segregate cohesive particles and to improve content uniformity,
- addition of lubricant and/or glidant and mixing the mixture to obtain a compression mixture,
- compressing the compression mixture to desired solid dosage form,
- optionally, applying a film coating.

### Detailed description of the invention

Herewith disclosed is a pharmaceutical formulation, comprising (a) as the active ingredient, rasagiline in the form of a pharmaceutically acceptable salt, (b) diluent, (c) lubricant and/or glidant and optionally (d) disintegrant, (e) binder, wherein the composition is not prepared by wet granulation process.

Also disclosed is a pharmaceutical formulation that does not contain antioxidants and/or acids. In particular, it is preferred that the pharmaceutical composition does not comprise an acid having a pKa value of less than 7. The pharmaceutical composition according to the invention does not comprise an acid selected from the group consisting of citric acid, tartaric acid, phosphoric acid, benzoic acid and maleic acid.

The pharmaceutical compositions of the present invention are solid pharmaceutical compositions. The compositions can be in the form of tablets, minitablets, microtablets, coated tablets, coated minitablets, coated microtablets, pills, lozenges, etc. The pharmaceutical compositions of the present invention are preferably suitable for oral application. The formulations of the present invention preferably have the form of tablets. The term "tablet" as used herein is intended to encompass compressed pharmaceutical dosage formulations of all shapes and sizes. Uncoated tablets are particularly preferred.

The pharmaceutical compositions of the present invention are preferably formulated in a unit dosage form, each dosage form containing about 0.1 to about 10 mg of rasagiline in the form of a pharmaceutically acceptable salt, preferably about 0.2 mg to about 2 mg of rasagiline in the form of a pharmaceutically acceptable salt, more preferably tablet comprising 0.5 mg, 1 mg or 2 mg of rasagiline in the form of a pharmaceutically acceptable salt. The amounts given are calculated on the content of rasagiline free base.

According to the present invention the tablets are prepared by direct compression. During our research it was found that the tablets prepared by direct compression had significantly higher storage stability than the tablets prepared by wet granulation. The increase of the impurities has been observed already prior the tablets have been subjected to accelerated stability conditions. In order to prevent the formation of impurities, the process for the manufacture of the pharmaceutical formulation should be performed without using solvents. It was surprisingly found by the present inventors that stable pharmaceutical formulations comprising rasagiline in the form of a pharmaceutically acceptable salt as the active ingredient, which exhibits high storage stability and excellent dose uniformity, can be prepared by a simple direct compression process if rasagiline in the form of pharmaceutically acceptable salt is first homogeneously mixed with certain excipients and then subjected to direct compression. The direct compression is preferably performed in the absence of any solvent. In view of the fact that the excipients used by the present inventors are commonly used for manufacturing tablets, the finding that they allow the production of stable rasagiline formulations with desired dose uniformity without any need for addition of acids or antioxidants or using wet granulation process was totally unexpected.

One of the most obvious advantages of direct compression is cost effectiveness. Savings can occur in a number of areas including reduced processing time and thus reduced labour costs, fewer manufacturing steps and pieces of equipment, less process validation, and a lower consumption of power.

The most significant advantages concerning the tablet quality are the processing without any need for moisture and heat that are inherent in most wet granulation procedures and the avoidance of high compaction pressures involved in producing tablets by slugging or roll compaction. Such a process can improve the stability of tablet formulations that contain a moisture-and temperature-sensitive drug.

The term "direct compression" refers to a process, wherein the various components of a tablet are blended, optionally milled, sieved and then compressed into tablets. The blending of the compounds may be achieved in one or more steps. For instance, the active ingredient may first be mixed with a part of diluent and this mixture can then be combined with a mixture of other ingredients. The whole process is preferably performed in the absence of a solvent.

The direct compression process for manufacturing the pharmaceutical composition according to the invention comprises:
- mixing rasagiline tartrate and at least one excipient or a mixture of excipients to form a powder mixture which can be optionally sieved before mixing to give a compression mixture;
- compressing the compression mixture to desired solid dosage form;
- optionally, applying a film coating.

In a more preferred embodiment of the present invention, the direct compression process for manufacturing the pharmaceutical composition according to the invention comprises:
- mixing rasagiline tartrate and a portion of diluent and/or further excipients and sieving the mixture,
- mixing the mixture with the main portion of diluent and/or further excipients in a mixer to obtain homogenized mixture,
- optionally dry sieving the mixture through a sieve in order to segregate cohesive particles and to improve content uniformity,
- addition of lubricant and/or glidant and mixing the mixture to obtain compression mixture,
- compressing the compression mixture to desired solid dosage form,
- optionally, applying a film coating.

In an even more preferred embodiment of the present invention, the direct compression process for manufacturing the pharmaceutical composition according to the invention comprises:
- mixing rasagiline tartrate and a portion of diluent in a weight/weight ratio from 1:5 to 1: 50, preferably from 1:10 to 1:30, more preferably from 1:15 to 1:25, and/or further excipients and sieving the mixture,
- mixing the above obtained mixture with the main portion of diluent and/or further excipients in a mixer to obtain a homogenized mixture,
- optionally dry sieving the mixture through a sieve in order to segregate cohesive particles and to improve content uniformity,
- addition of lubricant and/or glidant and mixing the mixture to obtain a compression mixture,
- compressing the compression mixture to desired solid dosage form,
- optionally, applying a film coating.

Also disclosed is a composition which can be prepared by dry granulation process.

The dry granulation process preferably comprises a process which comprises:
- granulating optionally sieved rasagiline pharmaceutically acceptable salt and at least one excipient or a mixture of excipients using processes known in the art as slugging and/or roller compaction;
- optionally milling and sieving the obtained slugs or compacts to obtain a granulate with desired particle size distribution;
- addition of excipient or a mixture of excipients to the granulate to give a compression mixture;
- compressing the compression mixture to the desired solid dosage form;
- optionally, applying a film coating,
or a process which comprises:
- granulating a portion of optionally sieved rasagiline pharmaceutically acceptable salt and at least one excipient or a mixture of excipients using processes known in the art as slugging and/or roller compaction;
- optionally milling and sieving the obtained slugs or compacts to obtain a granulate with desired particle size distribution;
- addition of the rest of the rasagiline pharmaceutically acceptable salt and excipient or a mixture of excipients to the granulate to give a compression mixture;
- compressing the compression mixture to the desired solid dosage form;

The mixing of rasagiline pharmaceutically acceptable salt and at least one excipient or a mixture of excipients may be effected in conventional devices used for mixing of powder, e.g. motionless (passive) mixers, fluidized bed, diffusion, biconic diffusion, biconic, tubular, cubic, planetary, Y-, V-shaped or high-shear mixers. The same mixing equipment may be used in the preparation of the compression mixture in direct compression. In another embodiment of the present invention, the same equipment may be used in the preparation of compression mixture with the prior step of a granulate preparation by a granulation as described by the terms "dry granulation".

Dry granulation can be performed by processes known in the art as slugging and/or roller compaction, the latter being preferred. Rasagiline pharmaceutically acceptable salt, optionally sieved to eliminate agglomerates, is usually mixed and dry-granulated with at least one excipient or a mixture of excipients into slugs/compacted material by using roller compactor or compression machine. The obtained slugs/compacted material is crushed and optionally sieved to obtain a uniform distribution of the granules of a dry granulate.

Pharmaceutically acceptable salts of rasagiline are e. g. disclosed in EP 436492, such as hydrochloride or tartrate, in EP 812190 such as mesylate, esylate and sulfate or in WO 2010/007181 such as rasagiline benzoate, rasagiline galactarate, rasagiline gluconate, rasagiline D-glucuronate, rasagiline tosylate, rasagiline phosphate, rasagiline maleate, rasagiline succinate, rasagiline acetate, rasagiline L-tartrate salt, rasagiline L-hemitartrate, rasagiline fumarate, rasagiline hydrochloride crystalline forms (Forms I and II), and rasagiline besylate. According to the invention rasagiline tartrate salt is used, more preferably rasagiline hemitartrate salt, most preferably rasagiline L-hemitartrate salt is used.

The pharmaceutical composition according to the invention comprises (b) at least one diluent, wherein the composition comprises a mixture of microcrystalline cellulose and modified starch as diluents and/or binder.

The starches are modified starches, such as pregelatinized starch, which is a type of modified starch that has been processed to render the starch more flowable and directly compressible. Partially or wholly pregelatinized starches can be used.

Low moisture diluents may be used, having loss on drying less than 5% w/w, preferably less than 2% w/w and most preferably less than 1.5% w/w, measured by halogen dryer, such as Mettler Toledo HR73 or R 83 or equivalent equipment.

Microcrystalline cellulose may be selected from microcrystalline cellulose with average particle size from 10 µm to 200 µm, preferably from 20 to 150 µm and moisture content ≤ 5%. Particularly, microcrystalline cellulose may be selected from microcrystalline cellulose with average particle size 20µm and a moisture content ≤ 5%, such as Avicel PH-105, microcrystalline cellulose with average particle size 50µm and a moisture content ≤ 5%, such as Avicel PH-101 or Vivapur 101, microcrystalline cellulose with average particle size 50µm and a moisture content ≤ 2%, such as Avicel PH-113, microcrystalline cellulose with average particle size 90 to 120 µm and a moisture content ≤ 5%, such as Avicel PH-102 or Vivapur 102, microcrystalline cellulose with average particle size 90 to 120 µm and a moisture content ≤ 1.5%, such as Avicel PH-112.

It has been surprisingly found that the use of particular diluents and/or binders, i. e. a mixture of microcrystalline cellulose and modified starch makes direct the compression process applicable for the manufacture of rasagiline tablets showing desired chemical stability, dose uniformity, weight variation and flow properties.

Modified starch is selected from modified starch with average particle size from 10 µm to 100 µm, preferably from 30 to 70 µm. Particularly, modified starch may be selected from modified starch with average particle size about 50µm, such as Starch 1500 and Starch 1500 LM.

The amount of microcrystalline cellulose in the composition is typically in the range of about 50% to about 95% by weight. Preferably, the amount of microcrystalline cellulose is about 60% to about 90%, more preferably about 70% to about 85%, by weight of the composition.

The amount of modified starch, in the composition is typically in the range of about 1% to about 40% by weight. Preferably the amount of starch is about 5% to about 30%, more preferably about 10% to about 20%, by weight of the composition.

The terms "average particle size" and "particle size distribution" as used herein refer to volume mean diameter of particles. The diameter and volume mean diameter can be determined by laser light scattering using e.g. a Malvern Mastersizer MS2000. Particle sizes are determined by measuring the angular distribution of laser light scattered by a homogeneous suspension of particles.

Particle size of excipients was determined by a Malvern Mastersizer MS2000, scirocco unit, wherein the sample was dispersed in air flow.

For the production of tablets of the present invention it is preferred that the particle size of all excipients is of the same particle size range in order to minimize the segregation of the component of the mixture. The average particle size of the excipients used are preferably in the range of 20 to 200 µm, more preferably in the range of 50 to 150 µm .The main advantages of the use of the exipients with a particle size of the same degree are a high content uniformity due to a low segregation tendency, an excellent compactability, leading to constant tablet hardness, a good flowability providing a high weight consistency at various tableting speeds.

In the tablets of the invention the diluent may at the same time serve as a binder and/or disintegrant.

The pharmaceutical formulations of the present invention may contain additional ingredients selected from a wide variety of excipients known in the art of pharmaceutical formulation, such as disintegrants, binders, lubricants, and glidants.

Suitable disintegrants may be selected from crospovidone, starch, maize starch, pregelatinized starch, sodium starch glycollate, hydroxypropyl starch, microcrystalline cellulose, sodium and/or calcium salts of carboxymethyl cellulose, cross-linked carboxymethylcellulose (e.g. croscarmellose sodium and/or croscarmellose calcium), polacrilin potassium, low substituted hydroxypropylcellulose, sodium and/or calcium alginate, docusate sodium, methylcellulose, agar, guar gum, chitosan, alginic acid or mixtures thereof. Preferably, disintegrants are selected from maize starch, pregelatinized starch, sodium starch glycolate, crospovidone, carboxymethylcellulose sodium, and croscarmelose sodium.

Suitable binders may be selected from povidone (polyvinilpyrrolidone), copovidone (vinylpyrrolidone-vinyl acetate copolymer), powdered cellulose, crystalline cellulose, microcrystalline cellulose, silicified microcrystalline cellulose, cellulose derivatives such as hydroxymethylcellulose, hydroxyethylcellulose, hydroxypropylcellulose, low substituted hydroxypropylcellulose and hydroxypropyl methylcellulose or other cellulose ethers, corn or potato or rice starch, pregelatinized starch, α-starch or dextrin, gum arabic, pullulan, polymethacrylates or mixture of binders. Preferably, the dry binders can be selected from copovidone, povidone, hydroxypropylcellulose, hydroxypropylmethylcellulose, microcrystalline cellulose, starch derivatives such as pregelatinized starch, low substituted hydroxypropylcellulose or mixtures thereof. More preferably, the pharmaceutical formulation of the present invention comprises at least one binder, selected from microcrystalline cellulose or starch or mixtures thereof. Microcrystalline cellulose and starches has simultaneously the role of a binder, a disintegrant and of a diluent and thus the use of an additional binder and/or disintegrant can be avoided if microcrystalline cellulose and/or starch is used in the tablets of the invention.

Lubricants and glidants e. g. are used in the formulations of the invention in the usual standard way.

Suitable lubricants may be selected from lubricants, selected from the group of metal salts of fatty acids with 12 to 20 carbon atoms such as magnesium, calcium, aluminium or zinc stearate, magnesium palmitate and magnesium oleate, hydrogenated vegetable oil, hydrogenated castor oil, talc, meads wax or spermaceti, boric acid, sodium stearyl fumarate, macrogols or mixtures thereof. Preferably, lubricants are selected from stearic acid, talc, sodium stearyl fumarate or mixtures thereof; most preferably stearic acid is used.

The pharmaceutical formulation of the present invention can furthermore comprise one or more glidants, selected from colloidal silica, talc and magnesium trisilicate, most preferably silica, colloidal anhydrous and talc is used.

If not specified otherwise, all percentages herein are by weight and based on the total weight of the tablet. The active ingredient is evenly distributed in a matrix formed by the other ingredients of the formulation. The tablets do preferably not have a layered structure and, as noted above, are preferably uncoated.

According to a more preferred embodiment, the pharmaceutical formulation of the present invention comprises:

| | |
|---|---|
| rasagiline tartrate: | 0.2-5%, more preferably 0.30-3%, most preferably 0.4-2.5%; |
| diluent: | 50-99%, more preferably 70-97%, most preferably 75-95%; |
| binder: | 0-30%, more preferably 0-20%, most preferably 0-15%; |
| disintegrant: | 0-30%, more preferably 0-20%, most preferably 0-15%; |
| lubricant: | 0.25-10%, more preferably 1-5%, most preferably 1.5-3%; and |
| glidant: | 0.25-10%, more preferably 1-5%, most preferably 1.5-3%, |

wherein the pharmaceutical formulation does not contain antioxidants and/or acids.

The content of rasagiline is based on the content of rasagiline in the form of pharmaceutically acceptable salt thereof.

In the preferred embodiment the formulation of the invention comprises 0.3 to 3% of rasagiline tartrate, 70 to 97 % by weight of a diluent selected from microcrystalline cellulose, 0 to 15 % by weight of a binder selected from pregelatinized starch or 0 to 5 % by weight of an disintegrant selected from croscarmellose sodium, and 0.25 to 5 % by weight of a lubricant, preferably selected from stearic acid, and 0.25 to 5 % by weight of a glidant, preferably selected from silica, colloidal anhydrous and talc.

In even more preferred embodiment the formulation of the invention comprises 0.3 to 3% of rasagiline tartrate, 70 to 85 % by weight of a diluent selected from microcrystalline cellulose, 10 to 20 % by weight of a binder selected from pregelatinized starch, and 1 to 5 % by weight of a lubricant, preferably selected from stearic acid, and 1 to 5 % by weight of a glidant, preferably selected from silica, colloidal anhydrous and talc.

In the state of the art it is stressed that, due to the low content of rasagiline salt in the composition, consistent dose uniformity is imperative and that such uniformity is hardly obtained by direct compression. We have unexpectedly found that, despite the low dose of the active compound in the tablet according to present invention, excellent dose uniformity is obtained, which is shown by analytical results of the content uniformity test where the relative standard deviation (RSD) is from 0.7 to 3 % and where the Acceptance Value (AV) is from 1 to 8. This can be attributed to the good flowability of the compression mixture, which therefore provides for a low weight variation of manufactured tablets, the RSD is equal to or less than 0.8 %, preferably less than 0.5% at various compression speeds.

The content uniformity of the tablets is characterized by the relative standard deviation (RSD) and Acceptance Value (AV), which are determined according to standard procedures as described in European Pharmacopoeia (2.9.40. Uniformity of Dosage Units).

The test for content uniformity of preparations presented in dosage units is based on the assay of the individual contents of active substance of a number of dosage units. The method used for the determination of assay was a reversed-phase isocratic HPLC method, using a 5 µm C18 column, or suitably validated alternative. The mobile phase is a mixture of ammonium dihydrogenphosphate and acetonitrile, with UV detection at 210 nm.

Hence, the pharmaceutical composition according to the invention is preferably characterized in that it exhibits a content uniformity of rasagiline with a relative standard deviation of less than 0.5 % to 3% or 0.7 to 3%, preferably 0.7 to 3.0% and an acceptance value of 1 to 8, determined in accordance with European Pharmacopoeia, 2.9.40.

In another embodiment, the pharmaceutical composition of the present invention comprises rasagiline pharmaceutically acceptable tartrate salt with average particle size in the range of 1 and 150 µm, preferably between 5 and 100 µm and most preferably between 8 and 50 µm. The average particle size is determined by laser method using a Malvern Mastersizer Apparatus.

In another embodiment the pharmaceutical composition of the present invention comprises rasagiline pharmaceutically acceptable tartrate salt with particle size distribution of d(0.1) from 0.1 to 20 µm, d(0.5) from 1 to 150 µm, d(0.9) from 10 to 300 µm, preferably d(0.1)from 0.5 to 10 µm, d(0.5) from 3 to 100 µm, d(0.9) from 15 to 150 µm, more preferably d(0.1)from 0.5 to 5 µm, d(0.5) from 4 to 70 µm, d(0.9) from 20 to 100 µm.

The terms "average particle size" and "particle size distribution" as used herein refer to volume mean diameter of particles. The diameter and volume mean diameter can be determined by laser light scattering using e.g. a Malvern Mastersizer Apparatus MS 2000. Particle sizes are determined by measuring the angular distribution of laser light scattered by a homogeneous suspension of particles. The particles to be subjected to the particle size measurement are first suspended in appropriate non-polar dispersant and then subjected to a size determination in a Malvern Mastersizer MS 2000 instrument. Usually, 100-800 mg of substance is dispersed in 5-10 mL of dispersant. Particle size of rasagiline pharmaceutically acceptable salt is determined by Malvern Mastersizer 2000 Hydro S. The particles to be subjected to the particle size measurement are first suspended in appropriate non-polar dispersant e.g. n-hexane.

The tablets obtained by above described procedure can be optionally coated with a water soluble film coating. The film coating can be applied onto the tablet cores by processes and equipment know in state of the art. The applied film coating can improve the smoothness of the tablet surface and/or can improve the stability of the rasagiline in the tablet by diminishing the permeation of moisture and/or oxygen into the tablet core. Film coating can be applied as an aqueous or organic dispersion or in a special embodiment as a dry coating procedure known from the state of the art e.g. from the review published by Sauer D. et all in Int. J. Pharm. 2013, vol. 457 (2), pages 488-502. The mentioned film coating is based on water soluble polymers selected from hypromellose having viscosity of 2 w/w% aqueous solution of less than 50 mPas measured by method described in Ph. Eur., polyvinyl alcohol, copolymers of polyethylene glycol and polyvinyl alcohol such as Kollicoat IR and/or Kollicoat Protect produced by BASF company, carboxymethyl cellulose, derivatives of poly(methacrylic acid) such as Eudragit E from Evonic or the like polymers. Additional excipient can be incorporated into such film coating selected from plasticizers, pigments and colorants, antitacking agents. Film coating can have a thickness of at least 1 µm, preferably in the range of 1 to 40 µm. The thickness is determined by measuring of the thickness of the coating using SEM photograph of a crossection of a coated tablet.

Plasticizers can be selected from polyethylene glycol having average molecular weight of 200 to 8,000, propylene glycol, esters of citric acid such as triacetyl citrate, butyl sebacate etc.

Pigments can be selected from metal oxides such as titanium and iron oxides or mixture thereof.

Antitacking agents can be selected from talc, colloidal silicon dioxide, glycerol monostearate and the like.

Also disclosed is a packaged pharmaceutical composition comprising the solid pharmaceutical composition described above, which is present in a low gas permeable primary packaging. The low gas permeable primary packaging may comprise materials such as aluminium or polychloro-3-fluoroethylene homopolymer/PVC laminate. Typically, the thickness of the packaging will be in the range of 10 to 40 µm for AI/AI blisters and 10 to 110 µm for AI-polychloro-3-fluoroethylene homopolymer/PVC laminate blisters. Optionally, the packaged pharmaceutical composition may further comprise a desiccant. The desiccant may be placed inside the packaging unit together with a dosage form such as a tablet and/or in the closure system and/or can be incorporated into the walls of the primary packaging unit. For example, the pharmaceutical composition can be packaged in containers made of glass or polymers, with or without desiccant.

The intermediary mixture obtained during the manufacturing process as well as the pharmaceutical composition and the final solid oral dosage form may be embedded in a gaseous mixture, wherein the oxygen may be present at a concentration of between approximately 0 % to 10 % (v/v), preferably at a concentration of between approximately 0 to 5 % (v/v), most preferably at a concentration of between approximately 0 to 2 % (v/v). Preferably nitrogen or argon can be used as inert gas atmosphere during the manufacturing process and in the packaging procedure, wherein nitrogen is especially preferred.

The optimization of tablet formulation is closely related to the intention to have the process of tablet manufacturing as simple as possible and to avoid laborious operations that may unnecessarily expose the material to be processed to heat or increased moisture. According to the present invention the use of solvents for granulation, which may cause degradation of rasagiline, consequently, avoided moisture conditions under which rasagiline is not stable; additionally, no drying was required during the process, therefore the use of elevated temperatures which may cause degradation was avoided. Moreover, by the present invention the simplest means of production of a pharmaceutical tablet is used, thus saving equipment, labor and energy costs.

The following examples illustrate the invention and are not intended to restrict the scope of the invention in any way.

### Examples

### Reference Examples 1 and 2

Composition of a tablet prepared according to EP 858328:

| Ingredients | Example 1 (mg/tbl) | Example 2 (mg/tbl) |
|---|---|---|
| Rasagiline mesylate | 1.56 | / |
| Rasagiline hemitartrate | / | 1.44 |
| Mannitol | 159.24 | 159.24 |
| Maize starch | 20.00 | 20.00 |
| Pregelatinized starch | 20.00 | 20.00 |
| Aerosil 200 | 1.20 | 1.20 |
| Talc | 4.00 | 4.00 |
| Stearic acid | 4.00 | 4.00 |

### Process:

Purified water was used as granulation liquid. A mixture of rasagiline mesylate or hemitartrate, mannitol, maize starch and Aerosil 200 was granulated with water in a high shear mixer and then dried in a fluid bed. After sieving the granulate through a 1.0 mm sieve, the granules were blended with pregelatinized starch and talc. The mixture was finally blended with stearic acid. The obtained mixture was compressed into tablets comprising 1.44 mg of rasagiline hemitartrate or 1.56 mg of rasagiline mesylate (equivalent to 1 mg of rasagiline base) on a rotary press machine with 12 x 8 mm rounded punches.

### Stability testing:

The tablets were stored in vials at 40°C/75%RH for 3 and 6 months. The known impurity A (1-aminoindane) and impurity B (1-indan-one), unidentified impurities and the sum of impurities were monitored by HPLC. The results were as follows:

| | Example 1 | | | Example 2 | | |
|---|---|---|---|---|---|---|
| Impurity | t=0 | 3 months at 40°C/75%RH | 6 months at 40°C/75%RH | t=0 | 3 months at 40°C/75%RH | 6 months at 40°C/75%RH |
| Impurity A | nd | 0.16% | 0.38% | 0.04 | 0.54% | 1.08% |
| Impurity B | nd | 0.10% | 0.23% | nd | 0.29% | 0.61% |
| Total unidentified | nd | 0.06% | 0.15% | nd | 0.31% | 0.43% |
| Total impurities | nd | 0.32% | 0.76% | 0.04 | 1.14% | 2.12% |

| | | | | | | |
|---|---|---|---|---|---|---|
| nd: not detected | | | | | | |

The method used for the determination of related substances of rasagiline was a reversed-phase gradient HPLC method, using a 3 µm C18 column, or suitably validated alternative. The mobile phase was a mixture of ammonium dihydrogenphosphate and acetonitrile, with UV detection at 210 nm.

As evident from the results of stability testing, the tablet according to Example 2 comprising rasagiline hemitartrate is much less stable compared to the tablet according to Example 1 comprising rasagiline mesylate. Thus, use of a wet granulation process and use of mannitol is not particularly suitable for manufacturing a stable pharmaceutical composition comprising rasagiline hemitartrate as the active ingredient.

### Example 3 and Reference Example 4

| Ingredients | Example 3* (mg/tbl) | Reference Example 4 (mg/tbl) |
|---|---|---|
| Rasagiline hemitartrate | 1.44 | 1.44 |
| Avicel PH 112 | 184.79 | 184.79 |
| Croscarmellose sodium | 5.00 | 5.00 |
| Aerosil 200 | 1.00 | 1.00 |
| Talc | 4.00 | 4.00 |
| Stearic acid | 4.00 | 4.00 |
| Process | Direct compression | Wet granulation |

| | | |
|---|---|---|
| not according to the invention | | |

### Process for Example 3:

Rasagiline hemitartrate, Aerosil 200, crosscarmellose sodium and part of Avicel (about 15 wt.-% of the whole Avicel quantity) were sieved through a 0.63 mm sieve to obtain a triturate mixture. The mixture was than blended with the remaining part of Avicel to obtain a homogenized mixture. The stearic acid and talc were then added and blended. The composition was compressed into tablets comprising 1.44 mg of rasagiline hemitartrate (equivalent to 1 mg of rasagiline base) in a rotary press machine with 12 x 8 mm rounded punches.

### Process for Reference Example 4:

Purified water was used as granulation liquid. A mixture of rasagiline hemitartrate, Avicel, croscarmellose sodium and Aerosil 200 was granulated with water in a high shear mixer and then dried in a fluid bed. After sieving the granulate through a 1.0 mm sieve, the granules were finally blended with stearic acid and talc. The composition was compressed into tablets comprising 1.44 mg of rasagiline hemitartrate (equivalent to 1 mg of rasagiline base) in a rotary press machine with 12 x 8 mm rounded punches.

### Stability testing:

The tablets were stored in vials at 40°C/75%RH for 3 and 6 months. The known impurity A (1-aminoindane) and impurity B (1-indan-one), unidentified impurities and the sum of impurities were monitored by HPLC. The results were as follows:

| | Example 3 | | | Reference Example 4 | | |
|---|---|---|---|---|---|---|
| Impurity | t=0 | 3 months at 40°C/75%RH | 6 months at 40°C/75%RH | t=0 | 3 months at 40°C/75%RH | 6 months at 40°C/75%RH |
| Impurity A | 0.02 | 0.24 | 0.48 | 0.03 | 0.38 | 0.76 |
| Impurity B | nd | 0.05 | 0.14 | 0.02 | 0.19 | 0.42 |
| Total unidentified | 0.00 | 0.03 | 0.11 | 0.00 | 0.24 | 0.37 |
| Total impurities | 0.02 | 0.32 | 0.73 | 0.05 | 0.81 | 1.55 |

| | | | | | | |
|---|---|---|---|---|---|---|
| nd: not detected | | | | | | |

Stability results for Reference Example 4 show that wet granulation is not a suitable method for preparation of a stable formulation of rasagiline hemitartrate, whereas the product prepared by direct compression exhibits excellent storage stability.

### IPC of the compression process:

| | | Example 3 | Example 4 |
|---|---|---|---|
| Mass of the tablets | Average (mg) | 200.4 | 201.8 |
| | Minimum(mg) | 198.8 | 201.0 |
| | Maximum (mg) | 201.8 | 202.8 |
| | RSD (%) | 0.39 | 0.30 |
| Hardness (N) | | 83 | 85 |
| Compression force (kN) | | 4.1 | 5.7 |

### Assay and content uniformity of the rasagiline in the tablets:

| | Example 3 | Example 4 |
|---|---|---|
| Assay of rasagiline base (%) | 97.2 | 98.6 |
| Content uniformity (AV) | 2.6 | 2.4 |

### Characteristics of rasagiline hemitartrate particles used in presented formulations:

| Average diameter (µm) | d(0.1) (µm) | d(0.5) (µm) | d(0.9) (µm) |
|---|---|---|---|
| 14.0 | 1.1 | 5.3 | 38 |

The average diameter of Avicel PH112 particles was about 100 µm.

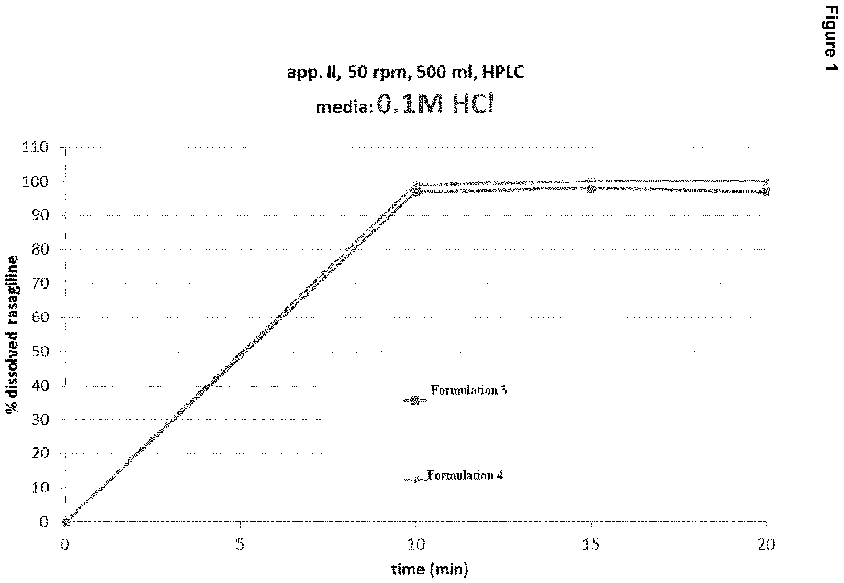

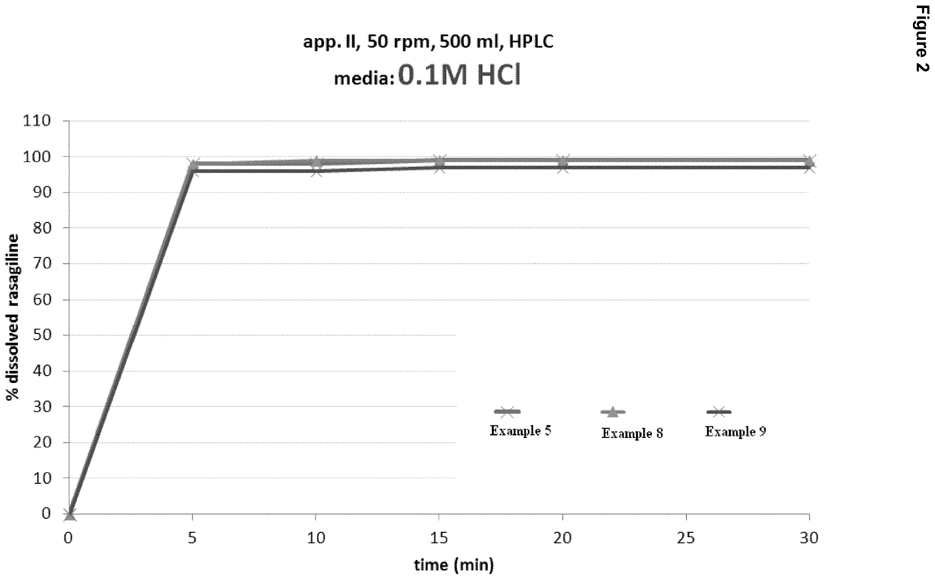

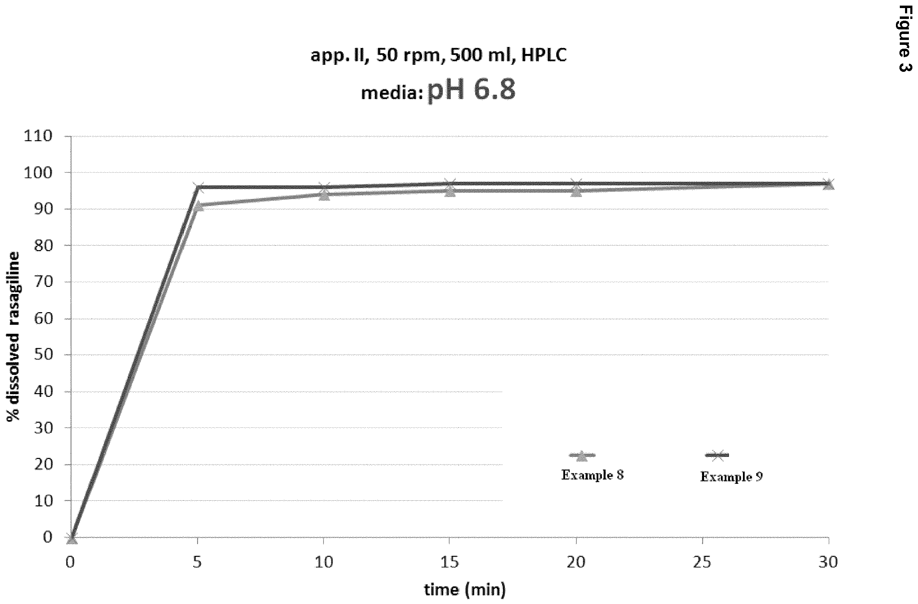

The dissolution profiles shown in Figures 1 to 3 were determined by a dissolution test performed on the tablets to be tested using Apparatus 2 as described in Chapter 711 (Dissolution) of the US Pharmacopeia under the following conditions:
Stirring speed: 50 rpm.
Temperature: 37°C ± 0,5°C
Dissolution medium: Phosphate buffer pH 6.8 or 0.1 M Hydrochloric acid
Medium volume: 500 ml, dissolution time: 30 min, sampling interval: 5 min

To establish the dissolution profile aliquots were taken at regular intervals of 5 minutes from the vessels and the concentration of the drug therein was determined by HPLC.

### Examples 5 to 9

| Ingredients | Example 5* (mg/tbl) | Example 6* (mg/tbl) | Example 7* (mg/tbl) | Example 8 (mg/tbl) | Example 9 (mg/tbl) |
|---|---|---|---|---|---|
| Rasagiline hemitartrate | 1.44 | 1.44 | 1.44 | 1.44 | 1.44 |
| Avicel PH 112 | 134.56 | 139.56 | / | / | / |
| Avicel PH 102 | / | / | 139.56 | 117.06 | 117.06 |
| Starch 1500 | / | / | / | 22.5 | / |
| Starch 1500 LM | / | / | / | / | 22.5 |
| Croscarmellose sodium | 5.00 | / | / | / | / |
| Aerosil 200 | 1.00 | 1.00 | 1.00 | 1.00 | 1.00 |
| Talc | 4.00 | 4.00 | 4.00 | 4.00 | 4.00 |
| Stearic acid | 4.00 | 4.00 | 4.00 | 4.00 | 4.00 |
| Mass of the tablet (mg) | 150.00 | 150.00 | 150.00 | 150.00 | 150.00 |
| Target hardness of the tablet (N) | 80 | 100-120 | 100-120 | 100-120 | 100-120 |
| Process | Direct compression | Direct compression | Direct compression | Direct compression | Direct compression |

| | | | | | |
|---|---|---|---|---|---|
| * not according to the invention | | | | | |

### Manufacturing procedure (direct compression):

Rasagiline hemitartrate with part of Avicel PH 112 or Avicel PH102 (weight ratio 1:20), Aerosil 200, croscarmelose sodium (if present in the formulation) and Starch 1500 or 1500 LM (if present in the formulation) were sieved through a 0.63 mm sieve to obtain a triturate mixture. The mixture was than blended with other parts of Avicel PH112 or Avicel PH102 to obtain a homogenized mixture. To the mixture stearic acid and talc were then added and blended. The composition was compressed into tablets comprising 1.44 mg of rasagiline hemitartrate (equivalent to 1 mg of rasagiline base) in a rotary press machine with 14 x 7 mm rounded punches.

### Stability testing:

The tablets were stored in vials at 40°C/75%RH for 1 and/or 3 months. The known impurity A (1-aminoindane) and impurity B (1-indan-one), unidentified impurities and the sum of impurities were monitored by HPLC. The results were as follows:

| | Example 5 | | | Example 6 | | Example 7 | | Example 8 | | Example 9 | |
|---|---|---|---|---|---|---|---|---|---|---|---|
| Impurity | t=0 | 1m | 3 m | t=0 | 1 m | t=0 | 1 m | t=0 | 1 m | t=0 | 1 m |
| Impurity A | 0.02 | 0.08 | 0.25 | n.d. | 0.05 | n.d. | 0.04 | n.d. | 0.05 | n.d. | 0.05 |
| Impurity B | n.d. | 0.02 | 0.06 | n.d. | 0.02 | n.d. | 0.03 | n.d. | 0.02 | n.d. | 0.02 |
| Total unidentified | n.d. | 0.04 | 0.03 | n.d. | n.d. | n.d. | 0.01 | n.d. | 0.01 | n.d. | n.d. |
| Total impurities | 0.02 | 0.14 | 0.34 | n.d. | 0.07 | n.d. | 0.08 | n.d. | 0.08 | n.d. | 0.07 |

| | | | | | | | | | | | |
|---|---|---|---|---|---|---|---|---|---|---|---|
| n.d. - not detected | | | | | | | | | | | |

### IPC of the rasagiline mixture for compression and of the compression process:

| | | Example 5 | Example 6 | Example 7 | Example 8 | Example 9 |
|---|---|---|---|---|---|---|
| Mass of the tablets | Average (mg) | 200.4 | 150.4 | 150.6 | 151.7 | 151.5 |
| | RSD (%) | 0.39 | 0.34 | 0.41 | 0.41 | 0.31 |
| Hardness of the tablets | Average (N) | 81 | 99 | 104 | 116 | 115 |
| | RSD (%) | 2.85 | 2.89 | 2.94 | 2.40 | 3.08 |
| Angle of repose (°) | | 38.0 | 40.7 | 38.0 | 38.7 | 37.8 |
| Haussner ratio | | 1.39 | 1.4 | 1.37 | 1.4 | 1.32 |

### Assay and content uniformity of the rasagiline in the tablets:

| | Example 5 | Example 6 | Example 7 | Example 8 | Example 9 |
|---|---|---|---|---|---|
| Assay of rasagiline (%) | 99.0 | 96.3 | 98.6 | 98.4 | 99.8 |
| RSD of assay of rasagiline base (%) | 1.1 | 2.7 | 2.3 | 2.1 | 1.9 |
| Content uniformity (AV10) | 2.6 | 5.5 | 5.1 | 4.9 | 4.5 |

### Characteristics of rasagiline hemitartrate particles used in presented formulations:

| Average diameter (µm) | d(0.1) (µm) | d(0.5) (µm) | d(0.9) (µm) |
|---|---|---|---|
| 31 | 1.5 | 6.6 | 53 |

## Claims

1. A pharmaceutical composition comprising (a), as the active ingredient, 0.2 to 5%, by weight of the composition, of rasagiline tartrate, (b) diluent, (c) lubricant and/or glidant and optionally (d) disintegrant and (e) binder, wherein the composition is prepared by direct compression, with the direct compression process comprising
- mixing rasagiline tartrate and at least one excipient or a mixture of excipients to form a compression mixture; and
- compressing the compression mixture to a solid dosage form,
wherein the composition is free of antioxidants and acids selected from the group consisting of citric acid, tartaric acid, phosphoric acid, benzoic acid and maleic acid,
and wherein the composition comprises a mixture of microcrystalline cellulose and modified starch as diluents and/or binder, wherein the modified starch has an average particle size from 10 um to 100 µm, wherein the average particle size refers to volume mean diameter determined by laser light scattering.

2. The pharmaceutical composition according to claim 1, wherein the rasagiline tartrate has a particle size distribution of d(0.1) from 0.1 to 20 µm, d(0.5) from 1 to 150 µm and d(0.9) from 10 to 300 µm, wherein the particle size distribution refers to volume mean diameter determined by laser light scattering.

3. The pharmaceutical composition according to any of the preceding claims, comprising (a) as the active ingredient rasagiline hemitartrate.

4. The pharmaceutical composition according to any of the preceding claims comprising, by weight of the composition:
| | |
|---|---|
| (a) rasagiline tartrate: | 0.2-5%, more preferably 0.30-3%, most preferably 0.4-2.5%; |
| (b) diluent: | 50-99%, more preferably 70-97%, most preferably 75-95%; |
| (e) binder: | 0-30%, more preferably 0-20%, most preferably 0-15%; |
| (d) disintegrant: | 0-30%, more preferably 0-20%, most preferably 0-15%; |
| (c)_lubricant: | 0.25-10%, more preferably 1-5%, most preferably 1.5-3%; |
| (c)_glidant: | 0.25-10%, more preferably 1-5%, most preferably 1.5-3%. |

5. The pharmaceutical composition according to any of the preceding claims, comprising 0.3 to 3% by weight of rasagiline tartrate, 70 to 97 % by weight of a diluent selected from microcrystalline cellulose, 0 to 15 % by weight of a binder selected from pregelatinized starch or 0 to 5 % by weight of a disintegrant, selected from crosscarmellose sodium, and 0.25 to 5 % by weight of a lubricant, preferably selected from stearic acid, and 0.25 to 5 % by weight of a glidant, preferably selected from silica, colloidal anhydrous and talc.

6. The pharmaceutical composition according to any of the preceding claims, comprising 0.3 to 3% by weight of rasagiline tartrate, 70 to 85 % by weight of a diluent selected from microcrystalline cellulose, 10 to 20 % by weight of a binder selected from pregelatinized starch, and 1 to 5 % by weight of a lubricant, preferably selected from stearic acid, and 1 to 5 % by weight of a glidant, preferably selected from silica, colloidal anhydrous and talc.

7. The pharmaceutical composition according to any of the preceding claims, wherein the modified starch has an average particle size from 30 µm to 70 µm, wherein the average particle size refers to volume mean diameter determined by laser light scattering.

8. The pharmaceutical composition according to claim 7, wherein the particle size of all excipients is of the same particle size range, with the average particle size ranging from of 20 to 200 µm, wherein the average particle size refers to volume mean diameter determined by laser light scattering using a Malvern Mastersizer MS2000, wherein the sample is dispersed in air flow

9. The pharmaceutical composition according to any one of claims 1 to 7, wherein the microcrystalline cellulose is selected from microcrystalline cellulose with average particle size from 10 µm to 200 µm, preferably from 20 to 150 µm and the modified starch is selected from modified starch with average particle size from 10 µm to 100 µm, preferably from 30 to 70 µm, wherein the average particle size refers to volume mean diameter determined by laser light scattering.

10. The pharmaceutical composition according to any of the preceding claims, wherein the direct compression process comprises
- mixing rasagiline tartrate and at least one excipient or a mixture of excipients to form a powder mixture which is sieved before mixing to give a compression mixture;
- compressing the compression mixture to desired solid dosage form;
- optionally applying a film coating.

11. The pharmaceutical composition according to any of the preceding claims, wherein the direct compression process comprises
- mixing rasagiline tartrate and a portion of diluent and/or further excipients and sieving the mixture,
- mixing the mixture with the main portion of diluent and/or further excipients in a mixer to obtain a homogenized mixture,
- optionally dry sieving the mixture through a sieve in order to segregate cohesive particles and to improve content uniformity,
- addition of lubricant and/or glidant and mixing the mixture to obtain a compression mixture,
- compressing the compression mixture to desired solid dosage form,
- optionally applying a film coating.

12. The pharmaceutical composition according to any of the preceding claims, wherein the direct compression process comprises
- mixing rasagiline tartrate and a portion of diluent in a weight/weight ratio from 1:5 to 1: 50, preferably from 1:10 to 1:30, more preferably from 1:15 to 1:25, and/or further excipients and sieving the mixture,
- mixing the above obtained mixture with the main portion of diluent and/or further excipients in a mixer to obtain a homogenized mixture,
- optionally dry sieving the mixture through a sieve in order to segregate cohesive particles and to improve content uniformity,
- addition of lubricant and/or glidant and mixing the mixture to obtain a compression mixture,
- compressing the compression mixture to desired solid dosage form,
- optionally applying a film coating.

13. A process for preparation of the pharmaceutical composition according to any of the preceding claims, which comprises
- mixing rasagiline tartrate and at least one excipient or a mixture of excipients to form a powder mixture which is optionally sieved before mixing to give a compression mixture;
- compressing the compression mixture to desired solid dosage form;
- optionally, applying a film coating.

14. The process according to claim 13, which comprises
- mixing rasagiline tartrate and a portion of diluent in a weight/weight ratio from 1:5 to 1: 50, preferably from 1:10 to 1:30, more preferably from 1:15 to 1:25, and/or further excipients and sieving the mixture,
- mixing the above obtained mixture with the main portion of diluent and/or further excipients in a mixer to obtain a homogenized mixture,
- optionally dry sieving the mixture through a sieve in order to segregate cohesive particles and to improve content uniformity,
- addition of lubricant and/or glidant and mixing the mixture to obtain a compression mixture,
- compressing the compression mixture to desired solid dosage form,
- optionally, applying a film coating.

## Patentansprüche

1. Pharmazeutische Zusammensetzung, die (a) als Wirkstoff bezogen auf das Gewicht der Zusammensetzung 0,2 bis 5 % Rasagilintartrat, (b) Verdünnungsmittel, (c) Schmiermittel und/oder Gleitmittel und gegebenenfalls (d) Sprengmittel und (e) Bindemittel enthält, wobei die Zusammensetzung durch Direktverpressung hergestellt ist, wobei bei der Direktverpressung
- Rasagilintartrat und mindestens ein Hilfsstoff oder eine Mischung von Hilfsstoffen gemischt werden, um eine Pressmischung herzustellen, und
- die Pressmischung zu einer festen Dosierform gepresst wird,
wobei die Zusammensetzung frei von Antioxidationsmitteln und Säuren ausgewählt aus der Gruppe bestehend aus Zitronensäure, Weinsäure, Phosphorsäure, Benzoesäure und Maleinsäure ist, und
wobei die Zusammensetzung als Verdünnungsmittel und/oder Bindemittel eine Mischung von mikrokristalliner Cellulose und modifizierter Stärke enthält, wobei die die modifizierte Stärke eine mittlere Partikelgröße von 10 µm bis 100 µm aufweist, wobei sich die mittlere Partikelgröße auf den durch Laserlichtstreuung bestimmten mittleren Volumendurchmesser bezieht.

2. Pharmazeutische Zusammensetzung nach Anspruch 1, bei der das Rasagilintartrat eine Partikelgrößenverteilung d(0,1) von 0,1 bis 20 µm, d(0,5) von 1 bis 150 µm und d(0,9) von 10 bis 300 µm aufweist, wobei sich die Partikelgrößenverteilung auf den durch Laserlichtstreuung bestimmten mittleren Volumendurchmesser bezieht.

3. Pharmazeutische Zusammensetzung nach einem der vorhergehenden Ansprüche, die (a) als Wirkstoff Rasagilinhemitartrat enthält.

4. Pharmazeutische Zusammensetzung nach einem der vorhergehenden Ansprüche, die bezogen auf das Gewicht der Zusammensetzung:
| | |
|---|---|
| (a) Rasagilintartrat: | 0,2 - 5 %, |
| | insbesondere 0,30 - 3 %, |
| | besonders bevorzugt 0,4 - 2,5 %, |
| (b) Verdünnungsmittel: | 50 - 99 %, |
| | insbesondere 70 - 97 %, |
| | besonders bevorzugt 75 - 95 %, |
| (e) Bindemittel: | 0 - 30 %, |
| | insbesondere 0 - 20 %, |
| | besonders bevorzugt 0 - 15 %, |
| (d) Sprengmittel: | 0 - 30 %, |
| | insbesondere 0 - 20 %, |
| | besonders bevorzugt 0 - 15 %, |
| (c) Schmiermittel: | 0,25 - 10 %, |
| | insbesondere 1 - 5 %, |
| | besonders bevorzugt 1,5 - 3 %, |
| (c) Gleitmittel: | 0,25 - 10 %, |
| | insbesondere 1 - 5 %, |
| | besonders bevorzugt 1,5 - 3 %, enthält. |

5. Pharmazeutische Zusammensetzung nach einem der vorhergehenden Ansprüche, die 0,3 bis 3 Gew.-% Rasagilintartrat, 70 bis 97 Gew.-% von einem Verdünnungsmittel, das aus mikrokristalliner Cellulose ausgewählt ist, 0 bis 15 Gew.-% von einem Bindemittel, das aus vorgelatinierter Stärke ausgewählt ist, oder 0 bis 5 Gew.-% von einem Sprengmittel, das aus Croscarmellose Natrium ausgewählt ist, und 0,25 bis 5 Gew.-% von einem Schmiermittel, das vorzugsweise aus Stearinsäure ausgewählt ist, und 0,25 bis 5 Gew.-% von einem Gleitmittel, das vorzugsweise aus kolloidalem wasserfreiem Siliciumdioxid und Talk ausgewählt ist, enthält.

6. Pharmazeutische Zusammensetzung nach einem der vorhergehenden Ansprüche, die 0,3 bis 3 Gew.-% Rasagilintartrat, 70 bis 85 Gew.-% von einem Verdünnungsmittel, das aus mikrokristalliner Cellulose ausgewählt ist, 10 bis 20 Gew.-% von einem Bindemittel, das aus vorgelatinierter Stärke ausgewählt ist, und 1 bis 5 Gew.-% von einem Schmiermittel, das vorzugsweise aus Stearinsäure ausgewählt ist, und 1 bis 5 Gew.-% von einem Gleitmittel, das vorzugsweise aus kolloidalem wasserfreiem Siliciumdioxid und Talk ausgewählt ist, enthält.

7. Pharmazeutische Zusammensetzung nach einem der vorhergehenden Ansprüche, bei der die modifizierte Stärke eine mittlere Partikelgröße von 30 µm bis 70 µm aufweist, wobei sich die mittlere Partikelgröße auf den durch Laserlichtstreuung bestimmten mittleren Volumendurchmesser bezieht.

8. Pharmazeutische Zusammensetzung nach Anspruch 7, bei der die Partikelgröße von allen Hilfsstoffen im gleichen Partikelgrößenbereich liegt, wobei die mittlere Partikelgröße von 20 bis 200 µm reicht, wobei sich die mittlere Partikelgröße auf den mittleren Volumendurchmesser bezieht, der durch Laserlichtstreuung unter Verwendung eines Malvern Mastersizers MS2000 bestimmt wird, wobei die Probe im Luftstrom dispergiert wird.

9. Pharmazeutische Zusammensetzung nach einem der Ansprüche 1 bis 7, bei der die mikrokristalline Cellulose ausgewählt ist aus mikrokristalliner Cellulose mit einer mittleren Partikelgröße von 10 µm bis 200 µm, vorzugsweise von 20 bis 150 µm, und die modifizierte Stärke ausgewählt ist aus modifizierter Stärke mit einer mittleren Partikelgröße von 10 µm bis 100 µm, vorzugsweise von 30 bis 70 µm, wobei sich die mittlere Partikelgröße auf den durch Laserlichtstreuung bestimmten mittleren Volumendurchmesser bezieht.

10. Pharmazeutische Zusammensetzung nach einem der vorhergehenden Ansprüche, bei der beim Direktverpressungsverfahren
- Rasagilintartrat und mindestens ein Hilfsstoff oder eine Mischung von Hilfsstoffen gemischt werden, um eine Pulvermischung herzustellen, die vor dem Mischen gesiebt wird, um eine Pressmischung zu erhalten,
- die Pressmischung zu einer gewünschten festen Dosierform gepresst wird und
- gegebenenfalls ein Filmüberzug aufgebracht wird.

11. Pharmazeutische Zusammensetzung nach einem der vorhergehenden Ansprüche, bei der beim Direktverpressungsverfahren
- Rasagilintartrat und ein Teil vom Verdünnungsmittel und/oder weiteren Hilfsstoffe gemischt werden und die Mischung gesiebt wird,
- die Mischung mit dem Hauptteil vom Verdünnungsmittel und/oder weiteren Hilfsstoffen in einem Mischer gemischt werden, um eine homogenisierte Mischung zu erhalten,
- gegebenenfalls die Mischung trocken durch ein Sieb gesiebt wird, um kohäsive Partikel abzutrennen und um die Einheitlichkeit des Gehalts zu verbessern,
- Schmiermittel und/oder Gleitmittel zugegeben werden und die Mischung gemischt wird, um eine Pressmischung zu erhalten,
- die Pressmischung zu einer gewünschten festen Dosierform gepresst wird und
- gegebenenfalls ein Filmüberzug aufgebracht wird.

12. Pharmazeutische Zusammensetzung nach einem der vorhergehenden Ansprüche, bei der beim Direktverpressungsverfahren
- Rasagilintartrat und ein Teil vom Verdünnungsmittel in einem Gewicht/Gewicht-Verhältnis von 1:5 bis 1:50, vorzugsweise von 1:10 bis 1:30, besonders bevorzugt 1:15 bis 1:25, und/oder weitere Hilfsstoffe gemischt werden und die Mischung gesiebt wird,
- die zuvor erhaltene Mischung mit dem Hauptteil vom Verdünnungsmittel und/oder weiteren Hilfsstoffen in einem Mischer gemischt werden, um eine homogenisierte Mischung zu erhalten,
- gegebenenfalls die Mischung trocken durch ein Sieb gesiebt wird, um kohäsive Partikel abzutrennen und um die Einheitlichkeit des Gehalts zu verbessern,
- Schmiermittel und/oder Gleitmittel zugegeben werden und die Mischung gemischt wird, um eine Pressmischung zu erhalten,
- die Pressmischung zu einer gewünschten festen Dosierform gepresst wird und
- gegebenenfalls ein Filmüberzug aufgebracht wird.

13. Verfahren zur Herstellung einer pharmazeutischen Zusammensetzung nach einem der vorhergehenden Ansprüche, bei dem
- Rasagilintartrat und mindestens ein Hilfsstoff oder eine Mischung von Hilfsstoffen gemischt werden, um eine Pulvermischung herzustellen, die gegebenenfalls vor dem Mischen gesiebt wird, um eine Pressmischung zu ergeben,
- die Pressmischung zu einer gewünschten festen Dosierform gepresst wird und
- gegebenenfalls ein Filmüberzug aufgebracht wird.

14. Verfahren nach Anspruch 13, bei dem
- Rasagilintartrat und ein Teil vom Verdünnungsmittel in einem Gewicht/Gewicht-Verhältnis von 1:5 bis 1:50, vorzugsweise von 1:10 bis 1:30, besonders bevorzugt von 1:15 bis 1:25, und/oder weitere Hilfsstoffe gemischt werden und die Mischung gesiebt wird,
- die zuvor erhaltene Mischung mit dem Hauptteil vom Verdünnungsmittel und/oder weiteren Hilfsstoffen in einem Mischer gemischt werden, um eine homogenisierte Mischung zu erhalten,
- gegebenenfalls die Mischung trocken durch ein Sieb gesiebt wird, um kohäsive Partikel abzutrennen und um die Einheitlichkeit des Inhalts zu verbessern,
- Schmiermittel und/oder Gleitmittel zugegeben werden und die Mischung gemischt wird, um eine Pressmischung zu erhalten,
- die Pressmischung zu einer gewünschten festen Dosierform gepresst wird und
- gegebenenfalls ein Filmüberzug aufgebracht wird.

## Revendications

1. Composition pharmaceutique comprenant (a), en tant que le composant actif, 0,2 à 5 %, en poids de la composition, de tartrate de rasagiline, (b) un diluant, (c) un lubrifiant et/ou agent de glissement, et en option (d) un désintégrant et (e) un liant, où la composition est préparée par compression directe, le procédé de compression direct comprenant les étapes consistant à
- mélanger du tartrate de rasagiline et au moins un excipient ou un mélange d'excipients, pour former un mélange de compression ; et
- comprimer le mélange de compression en une forme galénique solide,
où la composition est exempte d'antioxydants et d'acides choisis dans l'ensemble constitué par l'acide citrique, l'acide tartrique, l'acide phosphorique, l'acide benzoïque et l'acide maléique,
et où la composition comprend un mélange de cellulose microcristalline et d'amidon modifié en tant que diluants et/ou liant, l'amidon modifié ayant une taille moyenne de particule de 10 µm à 100 µm, la taille moyenne de particule se rapportant au diamètre moyen en volume déterminé par diffusion de lumière laser.

2. Composition pharmaceutique selon la revendication 1, dans laquelle le tartrate de rasagiline a une distribution de tailles de particules de d(0,1) de 0,1 à 20 µm, d(0,5) de 1 à 150 µm et d(0,9) de 10 à 300 µm, la distribution de tailles de particules se rapportant au diamètre moyen en volume déterminé par diffusion de lumière laser.

3. Composition pharmaceutique selon l'une quelconque des revendications précédentes, comprenant (a) en tant que le composant actif de l'hémitartrate de rasagiline.

4. Composition pharmaceutique selon l'une quelconque des revendications précédentes, comprenant, en poids de la composition :
| | |
|---|---|
| (a) tartrate de rasagiline : | 0,2-5 %, de façon plus particulièrement préférée 0,30-3 %, de façon tout particulièrement préférée 0,4-2,5 % ; |
| (b) diluant : | 50-99 %, de façon plus particulièrement préférée 70-97 %, de façon tout particulièrement préférée 75-95 % ; |
| (e) liant : | 0-30 %, de façon plus particulièrement préférée 0-20 %, de façon tout particulièrement préférée 0-15 % ; |
| (d) désintégrant : | 0-30 %, de façon plus particulièrement préférée 0-20 %, de façon tout particulièrement préférée 0-15 % ; |
| (c) lubrifiant : | 0,25-10 %, de façon plus particulièrement préférée 1-5 %, de façon tout particulièrement préférée 1,5-3 % ; |
| (c) agent de glissement : | 0,25-10 %, de façon plus particulièrement préférée 1-5 %, de façon tout particulièrement préférée 1,5-3 %. |

5. Composition pharmaceutique selon l'une quelconque des revendications précédentes, comprenant 0,3 à 3 % en poids de tartrate de rasagiline, 70 à 97 % en poids d'un diluant, choisi parmi de la cellulose microcristalline, 0 à 15 % en poids d'un liant, choisi parmi de l'amidon prégélatinisé ou 0 à 5 % en poids d'un désintégrant, choisi parmi de la croscarmellose sodique, et 0,25 à 5 % en poids d'un lubrifiant, choisi de préférence parmi de l'acide stéarique, et 0,25 à 5 % en poids d'un agent de glissement, choisi de préférence parmi de la silice colloïdale anhydre et du talc.

6. Composition pharmaceutique selon l'une quelconque des revendications précédentes, comprenant 0,3 à 3 % en poids de tartrate de rasagiline, 70 à 85 % en poids d'un diluant, choisi parmi de la cellulose microcristalline, 10 à 20 % en poids d'un liant, choisi parmi de l'amidon prégélatinisé, et 1 à 5 % en poids d'un lubrifiant, choisi de préférence parmi de l'acide stéarique, et 1 à 5 % en poids d'un agent de glissement, choisi de préférence parmi de la silice colloïdale anhydre et du talc.

7. Composition pharmaceutique selon l'une quelconque des revendications précédentes, dans laquelle l'amidon modifié a une taille moyenne de particule de 30 µm à 70 µm, la taille moyenne de particule se rapportant au diamètre moyen en volume déterminé par diffusion de lumière laser.

8. Composition pharmaceutique selon la revendication 7, dans laquelle la taille de particule de tous les excipients se situe dans la même plage de taille de particule, la taille moyenne de particule se situant dans la plage de 20 à 200 µm, la taille moyenne de particule se rapportant au diamètre moyen en volume déterminé par diffusion de lumière laser à l'aide d'un Mastersizer Malvern MS2000, dans lequel l'échantillon est dispersé dans un flux d'air.

9. Composition pharmaceutique selon l'une quelconque des revendications 1 à 7, dans laquelle la cellulose microcristalline est choisie parmi une cellulose microcristalline ayant une taille moyenne de particule de 10 µm à 200 µm, de préférence de 20 à 150 µm et l'amidon modifié est choisi parmi un amidon modifié ayant une taille moyenne de particule de 10 µm à 100 µm, de préférence de 30 à 70 µm, la taille moyenne de particule se rapportant au diamètre moyen en volume déterminé par diffusion de lumière laser.

10. Composition pharmaceutique selon l'une quelconque des revendications précédentes, dans laquelle le procédé de compression directe comprend les étapes consistant à
- mélanger du tartrate de rasagiline et au moins un excipient ou un mélange d'excipients, pour former un mélange pulvérulent qui est tamisé avant mélange pour donner un mélange de compression ;
- comprimer le mélange de compression en la forme galénique solide désirée ;
- en option appliquer un revêtement pelliculaire.

11. Composition pharmaceutique selon l'une quelconque des revendications précédentes, dans laquelle le procédé de compression directe comprend les étapes consistant à
- mélanger du tartrate de rasagiline et une partie du diluant et/ou des autres excipients et tamiser le mélange,
- homogénéiser dans un mélangeur le mélange avec la partie principale du diluant et/ou des autres excipients pour obtenir un mélange homogénéisé,
- en option tamiser à sec le mélange à travers un tamis afin de séparer les particules cohésives et d'améliorer l'uniformité du contenu,
- ajouter un lubrifiant et/ou un agent de glissement et homogénéiser le mélange pour obtenir un mélange de compression,
- comprimer le mélange de compression en la forme galénique solide désirée ;
- en option appliquer un revêtement pelliculaire.

12. Composition pharmaceutique selon l'une quelconque des revendications précédentes, dans laquelle le procédé de compression directe comprend les étapes consistant à
- mélanger du tartrate de rasagiline et une partie du diluant en un rapport poids/poids de 1:5 à 1:50, de préférence de 1:10 à 1:30, de façon plus particulièrement préférée de 1:15 à 1:25, et/ou des autres excipients et tamiser le mélange,
- homogénéiser dans un mélangeur le mélange obtenu ci-dessus avec la partie principale du diluant et/ou des autres excipients pour obtenir un mélange homogénéisé,
- en option tamiser à sec le mélange à travers un tamis afin de séparer les particules cohésives et d'améliorer l'uniformité du contenu,
- ajouter un lubrifiant et/ou un agent de glissement et homogénéiser le mélange pour obtenir un mélange de compression,
- comprimer le mélange de compression en la forme galénique solide désirée ;
- en option appliquer un revêtement pelliculaire.

13. Procédé pour la préparation de la composition pharmaceutique selon l'une quelconque des revendications précédentes, qui comprend les étapes consistant à :
- mélanger du tartrate de rasagiline et au moins un excipient ou un mélange d'excipients, pour former un mélange pulvérulent qui est en option tamisé avant mélange pour donner un mélange de compression ;
- comprimer le mélange de compression en la forme galénique solide désirée ;
- en option, appliquer un revêtement pelliculaire.

14. Procédé selon la revendication 13, qui comprend les étapes consistant à :
- mélanger du tartrate de rasagiline et une partie du diluant en un rapport poids/poids de 1:5 à 1:50, de préférence de 1:10 à 1:30, de façon plus particulièrement préférée de 1:15 à 1:25, et/ou des autres excipients et tamiser le mélange,
- homogénéiser dans un mélangeur le mélange obtenu ci-dessus avec la partie principale du diluant et/ou des autres excipients pour obtenir un mélange homogénéisé,
- en option tamiser à sec le mélange à travers un tamis afin de séparer les particules cohésives et d'améliorer l'uniformité du contenu,
- ajouter un lubrifiant et/ou un agent de glissement et homogénéiser le mélange pour obtenir un mélange de compression,
- comprimer le mélange de compression en la forme galénique solide désirée ;
- en option, appliquer un revêtement pelliculaire.
